(19) Europäisches Patentamt
European Patent Office
Office européen des brevets

(11) Publication number: 0 225 673 B1

(12) **EUROPEAN PATENT SPECIFICATION**

(45) Date of publication of the patent specification: 25.10.89

(51) Int. Cl.⁴: C07C 127/22

(21) Application number: 86202196.1

(22) Date of filing: 05.12.86

(54) **Process for the preparation of acyl ureas.**

(30) Priority: 09.12.85 GB 8530273

(43) Date of publication of application: 16.06.87 Bulletin 87/25

(45) Publication of the grant of the patent: 25.10.89 Bulletin 89/43

(84) Designated Contracting States: AT BE CH DE ES FR GB IT LI LU NL SE

(56) References cited:
EP-A- 0 086 281
GB-A- 1 324 293
US-A- 3 450 747

(73) Proprietor: SHELL INTERNATIONALE RESEARCH MAATSCHAPPIJ B.V., Carel van Bylandtlaan 30, NL-2596 HR Den Haag(NL)

(72) Inventor: Drent, Eit, Badhuisweg 3, NL-1031 CM Amsterdam(NL)
Inventor: Prillwitz, Peter Ernst, Badhuisweg 3, NL-1031 CM Amsterdam(NL)

(74) Representative: Aalbers, Onno et al, P.O. Box 302, NL-2501 CH The Hague(NL)

EP 0 225 673 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ACTORUM AG

## Description

The present invention relates to a process for the preparation of certain acyl ureas.

Acyl-substituted ureas have a wide variety of uses; in particular, certain acyl ureas are potent insecticides. Traditionally, such compounds have been made by reacting an amine with a carbonyl isocyanate or an amide with an isocyanate, as for example in UK Specification No. 1324293. This route proceeds in high yield, but involves a relatively large number of steps from commercially-available starting materials, and has the disadvantage of requiring an isocyanate intermediate. Many alternative methods of preparing isocyanates have been proposed, for example that of US Specification No. 3450747, but all suffer from serious drawbacks. The most widely used method involves phosgenation of the appropriate amide or amine; this process is efficient and proceeds in high yield, but phosgene is exceedingly toxic and accordingly very special handling precautions are necessary.

European Patent Application No. 86281A discloses a process for the preparation of carbamates from nitro compounds, hydroxy compounds and carbon monoxide, in the presence of a palladium-containing catalyst. Addition of a urea to the reaction mixture improves the reaction, the urea itself being converted into carbamate.

The Applicants have now found a phosgene-free route for the preparation of certain acyl ureas in one step from readily available starting materials.

Accordingly the present invention provides a process for the preparation of an N-aromatic-N′-acyl urea, which comprises reacting an aromatic nitro compound with a primary or secondary amide and carbon monoxide, in the presence of a catalyst comprising a Group VIII metal selected from iridium, ruthenium, rhodium, palladium and platinum, and a ligand which is an organo-phosphorus, organo-arsenic, organo-antimony or organo-nitrogen compound having a lone pair of electrons.

Throughout this Specification and claims, an acyl group should be understood to be an acyl group derived from an organic carboxylic acid.

The aromatic group in the aromatic nitro compound may be phenyl, naphthyl or heteroaryl, for example pyridyl, optionally substituted by one or more inert substituents, for example halogen atoms, alkyl, haloalkyl, alkoxy and haloalkoxy groups, preferably containing up to 4, especially 1 or 2, carbon atoms, and phenyl, phenoxy, pyridyloxy and pyridyl groups, themselves optionally substituted by one or more substituents selected from halogen atoms and alkyl, alkoxy, haloalkyl and haloalkoxy groups, preferably containing up to 4, especially 1 or 2, carbon atoms. Especially preferred are optionally-substituted nitrobenzenes.

The amide may be primary or secondary, for example of the general formula:

$R.NH.CO.R^1$ (I)

in which $R^1$ represents an optionally substituted alkyl, aryl or heteroaryl group and R represents an alkyl or optionally-substituted phenyl group, a group of formula $COR^2$ in which $R^2$ is an alkyl group or an optionally substituted phenyl group, or, preferably, a hydrogen atom. For example, $R^1$ may represent an alkyl group preferably having up to 10, especially up to 6, carbon atoms, optionally substituted by one or more substituents selected from halogen atoms, alkoxy and haloalkoxy groups preferably containing up to 4, especially 1 or 2, carbon atoms, and phenyl groups optionally substituted by one or more of the substituents listed above for the aromatic nitro compound; or $R^1$ represents a phenyl, naphthyl or pyridyl group optionally substituted by one or more of the substituents listed above for the aromatic nitro compound. Preferably $R^1$ represents an optionally substituted phenyl group.

Acyl ureas of major commercial significance are those having insecticidal activity, and thus the invention is of particular value when the nitro compound and amide are chosen to produce such a compound. Accordingly particularly preferred nitro compounds include 4-chloronitrobenzene; 4-(trifluoro-methoxy)nitrobenzene; 2,4-difluoro-3,5-dichloronitrobenzene; 4-(2-chloro-4-trifluoromethylphenoxy)nitrobenzene; 2-fluoro-4-(2-chloro-4-trifluoromethylphenoxy)nitrobenzene; and 3,5-dichloro-4-(3-chloro-5-trifluoromethylpyridyl-2-oxy)nitrobenzene. Although aliphatic amides such as acetamide may be used, optionally substituted benzamides are preferred. Especially useful acyl ureas are obtained using benzamide or halobenzamides carrying a fluorine and/or chlorine atom in the 2 and/or 6 position of the benzene ring, 2,6-difluorobenzamide being particularly preferred.

The catalyst used in the process of the invention is based on ruthenium, rhodium, palladium, iridium and/or platinum. The metal may be present as the element or a compound, but is preferably used as a salt or a complex. Suitable salts include salts derived from the oxy-acids of the metal, and also the metal halides, sulphates, nitrates, phosphates, hydroxides, oxides, and salts with organic acids, for example carboxylic acids having up to 20 carbon atoms, especially an alkanoic acid such as formic acid or acetic acid. Suitable complexes include those with carbon monoxide or acetylacetonates. Preferred metals are rhodium, ruthenium and, especially, palladium. Examples of convenient palladium salts include palladium chloride, palladium bromide, palladium iodide, sodium tetrachloropalladate, potassium tetrachloropalladate, potassium tetraiodopalladate, palladium formate, palladium acetate, palladium propionate, palladium isobutyrate and palladium acetylacetonate. Palladium acetate is especially preferred.

The amount of Group VIII metal used in the process according to the present invention is not critical, but is conveniently between 0.001%w and 10%w, in particular between 0.005%w and 3%w, calculated on the amount of aromatic nitro compound present in the reaction mixture.

The catalyst system also comprises a ligand which is an organo-phosphorus, organo-arsenic, organo-antimony or organo-nitrogen compound having a lone pair of electrons. The ligand may be monodentate or bidentate, that is it contains one or two hetero atoms through which it can coordinate with the catalyst metal to form a metal complex.

Examples of organo-phosphorus compounds which can conveniently be used include phosphites and tertiary phosphines of the general formula $P.OR^1.OR^2.OR^3$ or $PR^1R^2R^3$, wherein each of $R^1$, $R^2$ and $R^3$ independently represents an alkyl, cycloalkyl, aryl, alkaryl or aralkyl group having up to 10 carbon atoms, optionally substituted by one or more substituents selected from fluorine and chlorine atoms, alkoxy and aryloxy groups, in particular methoxy and phenoxy groups, cyano groups, and groups of formula $-PR^1R^2$; or $R^1$ and $R^2$ together with the interjacent heteroatom together represent a ring. Preferred monodentate organo-phosphorus compounds include trimethylphosphine, triethylphosphine, tri-n-butylphosphine and triphenylphosphine. Examples of bidentate phosphorus-containing ligands are 2-dimethyl-2-(methyldiphenylphosphino)-1,3-di(diphenylphosphino)propane, tetramethyl diphosphinoethane, tetramethyl diphosphinopropane, tetraethyl diphosphinoethane, tetrabutyl diphosphinoethane, dimethyl diethyl diphosphinoethane, tetraphenyl diphosphinoethane, tetraperfluorophenyl diphosphinoethane, tetraphenyl diphosphinopropane, tetraphenyl diphosphinobutane, dimethyl diphenyl diphosphinoethane, diethyl diphenyl diphosphinopropane, tetratolyl diphosphinoethane, ditolyl diphenyl diphosphinoethane, tetratrifluoromethyl diphosphinotetrafluoroethane, tetraphenyl diphosphinoethene and derivatives thereof and 1,2-bis-(diphenylphosphino)benzene and derivatives thereof. Preferred are tetraphenyldiphosphinobutane, tetraphenyl diphosphinoethane and tetraphenyl diphosphinopropane.

There may also be present in the reaction mixture as ligand one or more phosphorus V compounds which do not possess a lone pair of electrons. Such compounds include phosphine oxides, e.g. $OP.R^1R^2R^3$, and esters of various phosphorus acids, e.g. $OP.OR^1.(O)mR^2.(O)mR^3$, where $R^1$, $R^2$ and $R^3$ have the meanings given above, and each m independently is 0 or 1.

Examples of ligands containing an arsenic or antimony atom include the analogues of the phosphorus-containing compounds described above, for example tetraphenyl diarsaethane and tetraphenyl distibaethane.

Organo-nitrogen compounds which can conveniently be used include mono-, bi- or poly-cyclic systems containing one or more, especially 1 or 2, nitrogen atoms. Such cyclic organo-nitrogen compounds are preferably those of the formula:-

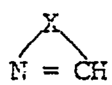 or 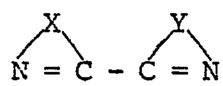

wherein X and Y, which may be the same or different, each represents a bridge group containing 3 or 4 atoms in the bridge of which at least 2 are carbon atoms, or can jointly form a multiple bridge, these ring systems preferably being aromatic. Specific examples of such compounds include pyrrole, pyridine, pyrimidine, phenanthroline, pyrazine, benztriazole, 2,2′-bipyridyl, 2,2′-biquinoline, bis-pyridylketone, bis-pyridylglyoxal, and their alkyl-substituted derivatives. Analogues containing other substituents, for example sulphonyl, carbonyl, alkoxy and halide moieties, may also be used. Preferred are 1,10-phenanthroline, 2,2′-bipyridyl, pyridine and alkyl-substituted pyridines such as the various picolines, e.g. alpha-picoline.

Preferred ligands are bidentate ligands containing nitrogen and/or phosphorus, with the nitrogen based ligands usually being the more effective, 2,2′-bipyridyl and 1,10 phenanthroline being especially preferred.

The ratio of the aromatic nitro compound to amide is not critical but preferably an excess of the aromatic nitro compound is used. Thus the molar ratio of nitro compound to amide may for example be 1:1 to 6:1, preferably 1:1 to 3:1. It is usually convenient to carry out the process in the presence of an inert diluent, for example an aromatic hydrocarbon such as benzene, toluene or xylene, a halogenated hydrocarbon such as a perfluoroalkane or chlorobenzene, a ketone, ester or ether, especially polyethers such as diglyme or tetraglyme, a tertiary amide such as N-methylpyrrolidone, or a sulphur-containing solvent such as sulpholane. Chlorobenzene or xylene is often the solvent of choice.

The reaction mixture may also, if desired, contain an acid as promotor. Suitable acids include carboxylic acids having 1 to 20 especially 1-10 carbon atoms, for example acetic acid, benzoic acid and substituted benzoic acids such as 2,4,5-trimethylbenzoic acid and 2,6-dichlorobenzoic acid; organic sulphonic acids; and inorganic acids such as sulphuric, phosphoric, perchloric, fluoroboric, fluorosilicic and fluorosulphonic acids. In general, it is found that weaker acids are more effective when a phosphorus ligand is present, whilst stronger acids are preferable in conjunction with nitrogen-containing ligands. Organic sulphonic acids, for example p-toluene sulphonic acid and trifluoromethyl sulphonic acid, are preferred. If present, the amount of acid in the reaction mixture is suitably between 0.01 and 150, especially 1 and 50, equivalents per gram atom of Group VIII metal.

The reaction mixture may also, if desired, contain a salt of copper, iron, manganese, chromium, vanadium or cobalt, either as well as or instead of an acid. Copper salts are preferred, with the anion suitably

being derived from the acids listed above as promotors. The amount of such metal salt is preferably in the range of 0.01 to 150, especially 1 to 50, calculated as gram atoms of metal per gram atom of Group VIII metal catalyst.

The process according to the present invention can be carried out conveniently at temperatures up to 250°C. Preferred are temperatures in the range of 80°C and 200°C, particularly 100°C to 150°C. The pressure is not critical, but the reaction is normally carried out at superatmospheric pressure, for example in the range 10-500, preferably 20 to 200, especially 50 to 90, atmospheres.

The process according to the invention usually proceeds with high selectivity to the desired product. It is suitably carried out in the absence of substantial quantities of hydroxylic compounds, such as water or alcohols, as such compounds lead to by-product formation. The presence of water can lead to formation of the disubstituted urea corresponding to the nitro compound; the amount of water which can be tolerated depends on the acceptable quantity of urea in the end product, but the presence of the quantities of water usually found in technical materials can normally be tolerated, and effects can be minimised by removing any water present azeotropically. Preferably the reaction mixture contains less than 5% by weight of hydroxylic compound.

The following Examples illustrate the invention. The reaction products were analysed by chromatography.

EXAMPLE 1

A 250 ml autoclave (Hastelloy C - Trade Mark) was charged with reactants 4-chloronitrobenzene (0.05mol) and 2,6-difluorobenzamide (0.0375mol), solvent p-xylene (50 ml), and catalyst components palladium acetate (0.5 mmol), p-toluene sulphonic acid (1mmol) and 2,2'-bipyridyl (10mmol). The autoclave was then pressurized with carbon monoxide (initial pressure 70 bar). The temperature was raised to 135°C and the reaction mixture was kept at this temperature for 5 hours. The reaction mixture was then allowed to cool, and the solid product filtered off and analysed. The product consisted of greater than 95% 1-(4-chlorophenyl)-3-(2,6-difluorobenzoyl)urea.

EXAMPLES 2 to 4

The general method of Example 1 was repeated using various reactants and catalyst components. Details and results are given in the Table below. DFBAM refers to 2,6-difluorobenzamide. Urea in the product is believed to result from side reactions involving water present in the technical materials used.

Table

| Example No. | Catalyst Components (mmol) | Reactants (mol) | Production Composition (%) |
|---|---|---|---|
| 2 | Palladium acetate (0.8) 2,2'-bipyridyl (20) 2,4,5-trichlorobenzene sulphonic acid (1) | 2,4-difluoro-3,5-dichloronitrobenzene (0.1) DFBAM (0.075) | 1-(2,4-difluoro-3,5-dichlorophenyl)-3-(2,6-difluorobenzoyl)-urea (77) 1,3-di(2,4-difluoro-3,5-dichlorophenyl)urea (8) DFBAM (9) |
| 3 | Palladium acetate (0.5) 2,2'-bipyridyl (10) p-toluene sulphonic acid (1) | 4-chloronitrobenzene (0.1) DFBAM (0.075) | 1-(4-chlorophenyl)-3-(2,6-difluorobenzoyl)urea (85) DFBAM (12) |
| 4 | Palladium acetate (0.5) 2,2'-bipyridyl (5) 2,4,5-trichlorophenyl sulphonic acid (1) | 2,4-difluoro-3,5-dichloronitrobenzene (0.1) DFBAM (0.075) | 1-(2,4-difluoro-3,5-dichlorophenyl)-3-(2,6-difluorobenzoyl)-urea(73) DFBAM (13) 1,3-di(2,4-difluoro3,5-dichlorophenyl)urea (11) |
| 5 | Palladium acetate (0.5) 2,2'-bipyridyl (10) p-toluene sulphonic acid (1) | 4-chloronitrobenzene (0.1) benzamide (0.075) | 1-(4-chlorophenyl)-3-benzoyl-urea (85) benzamide (10) 1,3-di(4-chlorophenyl)urea (5) |
| 6 | Palladium acetate (0.5) 2,4,5-trichlorobenzene sulphonic acid (1) phenanthroline (10) | 4-chloronitrobenzene (0.1) DFBAM (0.075) | 1-(4-chlorophenyl)-3-(2,6-difluorobenzoyl)urea (53) DFBAM (42) 1,3-di(4-chlorophenyl)urea (5) |

EXAMPLE 7

A 250 ml autoclave (Hastelloy C - Trade Mark) was charged with reactants 2,4-difluoro-3,5-dichloronitrobenzene (0.05mol) and 2,6-difluorobenzamide (0.0375mol), solvent p-xylene (50 ml), and catalyst components palladium acetate (0.1 mmol), copper (II) tosylate (0.12mmol) and 2,2′-bipyridyl (0.5mmol). The autoclave was then pressurized with carbon monoxide (initial pressure 70 bar). The temperature was raised to 135°C and the reaction mixture was kept at this temperature for 7.5 hours. The reaction mixture was then allowed to cool, and the solid product filtered off and analysed. The product consisted of 70% 1-(2,4-difluoro-3,5-dichlorophenyl)-3-(2,6-difluorobenzoyl)urea, 10% 1,3-di(2,4-difluoro-3,5-dichlorophenyl)urea and 20% 2,6-difluorobenzamide.

EXAMPLE 8

A 500 ml autoclave was charged with reactants 4-chloronitrobenzene (250 mmol) and 2,6-difluorobenzamide (180 mmol), solvent p-xylene (250 ml), and catalyst components palladium acetate (1.25 mmol), 2,2′-bipyridyl (25 mmol) and copper (II) tosylate (1.25 mmcl). The autoclave was then pressurised with carbon monoxide (initial pressure 50 bar), the temperature raised to 135°C and the reaction mixture held at this temperature for 12 hours. The reaction mixture was then allowed to cool, and the solid product filtered off and analysed, and found to contain 1-(4-chlorophenyl)-3-(2,6-difluorobenzoyl) urea in an amount representing a 83% yield (based on benzamide reactant).

EXAMPLE 9

Following a similar procedure to that of Example 8, but omitting the copper tosylate, the yield of urea product was 40% (based on benzamide).

## Claims

1. A process for the preparation of an N-aromatic-N′-acyl urea, which comprises reacting an aromatic nitro compound with a primary or secondary amide and carbon monoxide, in the presence of a catalyst comprising a Group VIII metal selected from iridium, ruthenium, rhodium, palladium and platinum and a ligand which is an organo-phosphorus, organo-arsenic, organo-antimony or organo-nitrogen compound having a lone pair of electrons.

2. A process as claimed in claim 1, in which the aromatic group in the aromatic nitro compound is phenyl, naphthyl or pyridyl optionally substituted by one or more substituents selected from halogen atoms,

alkyl, haloalkyl, alkoxy and haloalkoxy groups, and phenyl, phenoxy, pyridyl and pyridyloxy groups optionally substituted by one or more substituents selected from halogen atoms and alkyl, haloalkyl, alkoxy and haloalkoxy groups.

3. A process as claimed in claim 2, in which the aromatic nitro compound is selected from 4-chloronitrobenzene; 4-(trifluoromethoxy)nitrobenzene; 2,4-difluoro-3,5-dichloronitrobenzene; 4-(2-chloro-4-trifluoromethylphenoxy)nitrobenzene; 2-fluoro-4-(2-chloro-4-trifluoromethylphenoxy)nitrobenzene; and 3,5-dichloro-4-(3-chloro-5-trifluoromethylpyridyl-2-oxy)nitrobenzene.

4. A process as claimed in any one of claims 1 to 3, in which the amide has the formula:

$R.NH.CO.R^1$ (I)

in which R represents a hydrogen atom, an alkyl or optionally-substituted phenyl group, a group of formula $COR^2$ in which $R^2$ is an alkyl group or an optionally substituted phenyl group, and $R^1$ represents an optionally substituted alkyl, phenyl, naphthyl or pyridyl group, the optional substituents being selected from halogen atoms, alkyl, haloalkyl, alkoxy and haloalkoxy groups, and phenyl, phenoxy, pyridyl and pyridyloxy groups optionally substituted by one or more substituents selected from halogen atoms and alkyl, haloalkyl, alkoxy and haloalkoxy groups.

5. A process as claimed in claim 4, in which the amide is benzamide or a halobenzamide carrying a fluorine and/or chlorine atom in the 2 and/or 6 position of the benzene ring.

6. A process as claimed in any one of claims 1 to 5, in which the Group VIII metal is palladium.

7. A process as claimed in claim 6 in which the palladium is present in the form of a salt of an alkanoic acid.

8. A process as claimed in any one of claim 1-7, in which the ligand is a bidentate ligand containing nitrogen or phosphorus.

9. A process as claimed in claim 8 wherein the ligand is a nitrogen heterocycle of formula

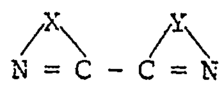

wherein X and Y, which may be the same or different, each represents a bridge group containing 3 or 4 atoms in the bridge at least 2 of which are carbon atoms, or can jointly form a multiple bridge, the ring systems thus formed being aromatic.

10. A process as claimed in claim 9 wherein the ligand is 2,2'-bipyridyl or 1,10-phenanthroline.

11. A process as claimed in any one of claims 1-10 wherein the reaction is carried out in the presence of an acid promotor.

12. A process as claimed in claim 11 wherein the acid promotor is a carboxylic acid, optionally substituted benzoic acid, an organic sulphonic acid, or an inorganic acid.

13. A prooess as claimed in claim 12 wherein the acid promotor is p-toluene sulphonic acid or trifluoromethyl sulphonic acid.

14. A process as claimed in any one of claims 1-13 wherein the reaction is carried out in the presence of a salt of copper, iron, manganese, chromium, vanadium, or cobalt.

15. A process as claimed in claim 14 wherein the reaction is carried out in the presence of a salt of copper with an acid as claimed in claim 12 or 13.

16. A process as claimed in any one of claims 1-15, carried out at a temperature in the range of 80° to 200°C and a pressure in the range of 10 to 500 atmospheres.

**Patentansprüche**

1. Verfahren zur Herstellung eines N-aromatischen-N'-Acylharnstoffes, welches das Umsetzen einer aromatischen Nitroverbindung mit einem primären oder sekundären Amid und Kohlenmonoxid beinhaltet, in Gegenwart eines Katalysators, welcher sich aus einem Gruppe-VIII-Metall, ausgewählt von Iridium, Ruthenium, Rhodium, Palladium und Platin, und einem Liganden, welcher eine Organo-Phosphor-, Organo-Arsen-, Organo-Antimon- oder Organo-Stickstoffverbindung mit einem einsamen Elektronenpaar ist, zusammensetzt.

2. Verfahren nach Anspruch 1, in welchem die aromatische Gruppe in der aromatischen Nitroverbindung Phenyl, Naphthyl oder Pyridyl ist, wahlweise substituiert durch einen oder mehrere Substituenten, ausgewählt von Halogenatomen, Alkyl-, Halogenalkyl-, Alkoxy- und Halogenalkoxygruppen, und Phenyl-, Phenoxy-, Pyridyl- und Pyridyloxygruppen, wahlweise substituiert durch einen oder mehrere Substituenten, ausgewählt von Halogenatomen und Alkyl-, Halogenalkyl-, Alkoxy- und Halogenalkoxygruppen.

3. Verfahren nach Anspruch 2, in welchem die aromatische Nitroverbindung von 4-Chlornitrobenzol; 4-(Trifluormethoxy)nitrobenzol; 2,4-Difluor-3,5-dichlornitrobenzol; 4-(2-Chlor-4-trifluormethylphenoxy)nitrobenzol; 2-Fluor-4-(2-chlor-4-trifluormethylphenoxy)nitrobenzol und 3,5-Dichlor-4-(3-chlor-5-trifluormethylpyridyl-2-oxy)nitrobenzol ausgewählt ist.

4. Verfahren nach einem der Ansprüche 1 bis 3, in welchem das Amid die Formel:

R.NH.CO.$R^1$ (I)
besitzt,
worin R ein Wasserstoffatom, eine Alkyl- oder wahlweise substituierte Phenylgruppe, eine Gruppe der Formel $COR^2$, in welcher $R^2$ eine Alkylgruppe oder eine wahlweise substituierte Phenylgruppe ist, darstellt, und $R^1$ für eine wahlweise substituierte Alkyl-, Phenyl-, Naphthyl- oder Pyridylgruppe steht, wobei die fakultativen Substituenten von Halogenatomen, Alkyl-, Halogenalkyl-, Alkoxy- und Halogenalkoxy-Gruppen, und Phenyl-, Phenoxy-, Pyridyl- und Pyridyloxygruppen, wahlweise substituiert durch einen oder mehrere Substituenten, ausgewählt von Halogenatomen und Alkyl-, Halogenalkyl-, Alkoxy- und Halogenalkoxygruppen, ausgewählt sind.

5. Verfahren nach Anspruch 4, in welchem das Amid Benzamid oder ein Halogenbenzamid ist, welches ein Fluor- und/oder Chloratom in der 2- und/oder 6-Stellung des Benzolringes besitzt.

6. Verfahren nach einem der Ansprüche 1 bis 5, worin das Gruppe-VIII-Metall Palladium ist.

7. Verfahren nach Anspruch 6, in welchem das Palladium in Form eines Salzes einer Alkansäure vorliegt.

8. Verfahren nach einem der Ansprüche 1 bis 7, in welchem der Ligand ein 2zähniger, Stickstoff- oder Phosphor-hältiger Ligand ist.

9. Verfahren nach Anspruch 8, worin der Ligand ein Stickstoff-Heterocyklus der Formel

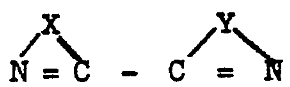

ist, worin X und Y, welche gleich oder verschieden sein können, jeweils eine Brückengruppe mit 3 oder 4 Atomen in der Brücke darstellen, wovon mindestens 2 Kohlenstoffatome sind, oder sie können gemeinsam eine Mehrfachbrücke bilden, wobei das so gebildete Ringsystem aromatisch ist.

10. Verfahren nach Anspruch 9, worin der Ligand 2,2'-Bipyridyl oder 1,10-Phenanthrolin ist.

11. Verfahren nach einem der Ansprüche 1 bis 10, worin die Reaktion in Gegenwart eines Säurepromotors ausgeführt wird.

12. Verfahren nach Anspruch 11, worin der Säurepromotor eine Carbonsäure, wahlweise substituierte Benzoesäure, eine organische Sulfonsäure oder eine anorganische Säure ist.

13. Verfahren nach Anspruch 12, worin der Säurepromotor p-Toluolsulfonsäure oder Trifluormethylsulfonsäure ist.

14. Verfahren nach einem der Ansprüche 1 bis 13, worin die Reaktion in Gegenwart eines Salzes von Kupfer, Eisen, Mangan, Chrom, Vanadium oder Kobalt ausgeführt wird.

15. Verfahren nach Anspruch 14, worin die Reaktion in Gegenwart eines Salzes von Kupfer mit einer Säure nach Anspruch 12 oder 13 ausgeführt wird.

16. Verfahren nach einem der Ansprüche 1 bis 15, ausgeführt bei einer Temperatur im Bereich von 80° bis 200°C und einem Druck im Bereich von 10 bis 500 Atmosphären.

**Revendications**

1. Un procédé pour la préparation d'une N-aromatique-N'-acylurée, qui comprend la réaction d'un composé nitro-aromatique avec un amide primaire ou secondaire et de l'oxyde de carbone, en présence d'un catalyseur comprenant un métal du groupe VIII choisi parmi l'iridium, le ruthénium, le rhodium, le palladium et le platine et un ligand qui est un composé organophosphore, organo-arsenic, organo-antimoine ou organo-azote ayant un doublet électronique libre.

2. Un procédé selon la revendication 1, dans lequel le groupe aromatique dans le composé nitro-aromatique est un groupe phényle, naphtyle ou pyridyle éventuellement substitué par un ou plusieurs substituants choisis parmi des atomes d'halogènes, des groupes alcoyle, haloalcoyle, alcoxy et haloalcoxy et des groupes phényle, phénoxy, pyridyle et pyridyloxy éventuellement substitués par un ou plusieurs substituants choisis parmi des atomes d'halogènes et des groupes alcoyle, haloalcoyle, alcoxy et haloalcoxy.

3. Un procédé selon la revendication 2, dans lequel le composé nitro-aromatique est choisi parmi le 4-chloronitrobenzène, le 4-(trifluorométhoxy)nitrobenzène, le 2,4-difluoro-3,5-dichloronitrobenzène, le 4-(2-chloro-4-trifluorométhylphénoxy)nitrobenzène, le 2-fluoro-4-(2-chloro-4-trifluorométhylphénoxy)nitrobenzène et le 3,5-dichloro-4-(3-chloro-5-trifluorométhylpyridyl-2-oxy)nitrobenzène.

4. Un procédé selon l'une quelconque des revendications 1 à 3, dans lequel l'amide a la formule:
R.NH.CO.$R^1$ (I)
dans laquelle R représente un atome d'hydrogène, un groupe alcoyle ou un groupe phényle éventuellement substitué, un groupe de formule $COR^2$ où $R^2$ est un groupe alcoyle ou un groupe phényle éventuellement substitué et $R^1$ représente un groupe alcoyle, phényle, naphtyle ou pyridyle éventuellement substitué, les substituants éventuels étant choisis parmi des atomes d'halogènes, des groupes alcoyle, haloalcoyle, alcoxy et haloalcoxy et des groupes phényle, phénoxy, pyridyle et pyridyloxy éventuellement substitués par un ou plusieurs substituants choisis parmi des atomes d'halogènes et des groupes alcoyle, haloalcoyle, alcoxy et haloalcoxy.

5. Un procédé selon la revendication 4, dans lequel l'amide est le benzamide ou un halobenzamide portant un atome de fluor et/ou un atome de chlore dans la position 2 et/ou la position 6 du noyau benzénique.

6. Un procédé selon l'une quelconque des revendications 1 à 5, dans lequel le métal du groupe VIII est du palladium.

7. Un procédé selon la revendication 6, dans lequel le palladium est présent sous la forme d'un sel d'un acide alcanoïque.

8. Un procédé selon l'une quelconque des revendications 1 à 7, dans lequel le ligand est un ligand bidenté contenant de l'azote ou du phosphore.

9. Un procécé selon la revendication 8, dans lequel le ligand est un hétérocycle azoté de formule

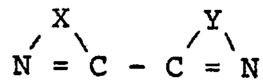

où X et Y, qui peuvent être identiques ou différents, représentent chacun un groupe formant pont contenant 3 ou 4 atomes dans le pont dont au moins 2 sont des atomes de carbone, ou peuvent former ensemble un pont multiple, le système cyclique ainsi formé étant aromatique.

10. Un procédé selon la revendication 9, dans lequel le ligand est le 2,2'-bipyridyle ou la 1,10-phénanthroline.

11. Un procédé selon l'une quelconque des revendications 1 à 10, dans lequel la réaction est conduite en présence d'un promoteur acide.

12. Un procédé selon la revendication 11, dans lequel le promoteur acide est un acide carboxylique, un acide benzoïque éventuellement substitué, un acide sulfonique organique ou un acide inorganique.

13. Un procédé selon la revendication 12, dans lequel le promoteur acide est l'acide p-toluène sulfonique ou l'acide trifluorométhyl sulfonique.

14. Un procédé selon l'une quelconque des revendications 1 à 13, dans lequel la réaction est conduite en présence d'un sel de cuivre, de fer, de manganèse, de chrome, de vanadium ou de cobalt.

15. Un procédé selon la revendication 14, dans lequel la réaction est conduite en présence d'un sel de cuivre avec un acide tel que revendiqué dans la revendication 12 ou 13.

16. Un procédé selon l'une quelconque des revendications 1 à 15, mis en œuvre à une température dans l'intervalle de 80° à 200°C et à une pression comprise dans l'intervalle de 10 à 500 atmosphères.